# EUROPEAN PATENT APPLICATION

(11) **EP 4 696 676 A1**
(43) Date of publication of application: **18.02.2026**
(21) Application number: 24788715.1
(22) Date of filing: 09.04.2024
(51) Int. Cl.: C07C 317/24, A01N 25/04, A01N 31/06, A01P 3/00

(54) **PHENYLCYCLOHEXANONE COMPOUND AND USE THEREFOR**

(30) Priority: 10.04.2023 JP 2023063233
(71) Applicant: Sumitomo Chemical Company, Limited, Tokyo 103-6020 (JP)
(72) Inventor: MAEHATA, Ryota, Takarazuka-shi, Hyogo 665-8555 (JP)
(74) Representative: Vossius & Partner Patentanwälte Rechtsanwälte mbB
(86) International application number: PCT/JP2024/014380
(87) International publication number: WO 2024/214691

(57) **Abstract**

The present invention provides a compound having excellent control efficacy against plant diseases. A compound represented by formula (A) 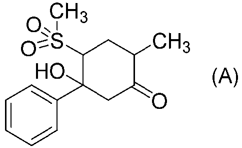 can be used for controlling plant diseases.

## Description

### TECHNICAL FIELD

This application claims the priority to and the benefit of Japanese Patent Application No. 2023-063233 filed on April 10, 2023, the entire contents of which are incorporated herein by reference.

The present invention relates to a phenylcyclohexanone compound and use thereof.

### BACKGROUND ART

Non-Patent Document 1 discloses compounds having control effects against plant diseases.

### CITATION LIST

### NON-PATENT DOCUMENT

Non-Patent Document 1: The Pesticide Manual-18th Edition (published by BCPC); ISBN 978-1-9998966-1-4

### SUMMARY OF THE INVENTION

### PROBLEMS TO BE SOLVED BY THE INVENTION

An object of the present invention is to provide a compound having excellent control efficacy against plant diseases.

### MEANS TO SOLVE PROBLEMS

The present inventor has studied the above problems. As a result, he has found that a compound represented by the following formula (A) has excellent control efficacy against plant diseases, and completed the present invention.

Namely, the present invention provides the followings.
[1] A compound represented by formula (A) 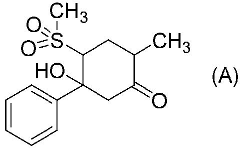 (hereinafter referred to as "Present compound" or "Compound of the present invention").
[2] A composition for controlling a plant disease comprising the compound according to [1] and an inert carrier (hereinafter referred to as "Present composition for controlling a plant disease" or "Composition for controlling a plant disease of the present invention").
[3] A method for controlling a plant disease which comprises applying the compound according to [1] or the composition for controlling a plant disease according to [2] to a plant or soil for cultivating a plant (hereinafter referred to as "Present method for controlling a plant disease" or "Method for controlling a plant disease of the present invention ").

### EFFECT OF INVENTION

The Present compound has excellent control efficacy against plant diseases, and is useful as an active ingredient of a composition for controlling plant diseases.

### MODE FOR CARRYING OUT THE INVENTION

The Present compound has two or more stereoisomers. Examples of the stereoisomers include enantiomers and diastereomers. The Present compound encompasses each stereoisomer and mixtures of stereoisomers at any ratio.

Examples of the stereoisomers include a compound represented by formula (1) 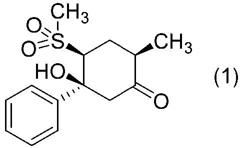 and a compound represented by formula (2) 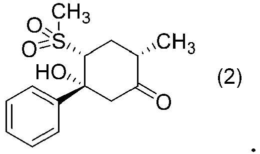 The compound represented by formula (1) and the compound represented by formula (2) are enantiomers with each other.

Aspects of the Present compound include the following compounds.

The compound represented by formula (1).

The compound represented by formula (2).

A compound represented by formula (3) 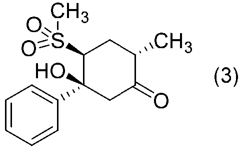 and an enantiomer of said compound.

A compound represented by formula (4) 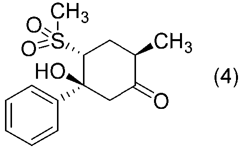 and an enantiomer of said compound.

A compound represented by formula (5) 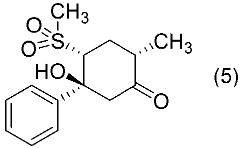 and an enantiomer of said compound.

A mixture of stereoisomers comprising a larger amount of the compound represented by formula (1) than the amount of any other stereoisomer.

A mixture of stereoisomers comprising a larger amount of the compound represented by formula (2) than the amount of any other stereoisomer.

A mixture of stereoisomers comprising the compound represented by formula (1) and the compound represented by formula (2) at a ratio of 1:1.

A mixture of stereoisomers comprising the compound represented by formula (1) and the compound represented by formula (2) at a ratio of 1:1, and the amount of the compound represented by formula (1) is larger than the amount of any other stereoisomer except for the compound represented by formula (1) and the compound represented by formula (2).

The Present composition for controlling a plant disease usually comprises the Present compound and an inert carrier. The Present composition for controlling a plant disease is usually prepared by mixing the Present compound with inert carrier(s) such as solid carrier(s) and liquid carrier(s), and as needed, adding surfactant(s) and/or other auxiliary agent(s) for formulation thereto to be formulated into a wettable powder, a granular wettable powder, a flowable, a granule, a dry flowable, an emulsifiable concentrate, a microcapsule, or the like to be used. These formulations usually comprise 0.0001%~99% by weight of the Present compound.

Examples of the solid carrier to be used in formulation include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

The Present compound can control plant diseases caused by plant pathogenic microorganisms such as fungi, Oomycete, Phytomyxea, and bacteria. Examples of the fungi include Ascomycota, Basidiomycota, Blastocladiomycota, Chytridiomycota, Mucoromycota, and Olpidiomycota. Specific examples thereof include the followings. The scientific name of plant pathogenic microorganism which causes each disease is shown in parentheses.

Rice diseases:
blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight (Trichoderma viride and Rhizopus oryzae), pseudo sheath blight (Waitea circinate, Ceratobasidium setariae, and Thanatephorus cucumeris), Rice false smut (Ustilaginoidea virens), and Kernel smut (Tilletia horrida and Tilletia barclayana);

Wheat diseases:
powdery mildew (Blumeria graminis), fusarium blight (Fusarium graminearum, Fusarium avenaceum, Fusarium culmorum, and Microdochium nivale), stripe rust (Puccinia striiformis), stem rust (Puccinia graminis), leaf rust (Puccinia recondita), snow mould (Microdochium nivale and Microdochium majus), typhula snow blight (Typhula incarnata and Typhula ishikariensis), Sclerotinia snow blight (Sclerotinia borealis), Browning root rot (Pythium spp.), loose smut (Ustilago tritici), stinking smut (Tilletia caries and Tilletia controversa), eyespot (Pseudocercosporella herpotrichoides), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graminis-tritici);

Barley diseases:
powdery mildew (Blumeria graminis), fusarium head blight (Fusarium graminearum, Fusarium avenaceum, Fusarium culmorum, and Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);

Corn diseases:
rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis and Stenocarpella macrospora), stalk rot (Fusarium graminearum, Fusarium verticilioides, and Colletotrichum graminicola), smut (Ustilago maydis), Physoderma brown spot and Physoderma stalk rot (Physoderma maydis), and tar spot disease (Phyllachora maydis);

Cotton diseases:
anthracnose (Colletotrichum gossypii), grey mildew (Ramularia areola), alternaria leaf spot (Alternaria macrospora and Alternaria gossypii), and black root rot (Thielaviopsis basicola);

Coffee diseases:
rust (Hemileia vastatrix) and leaf spot (Cercospora coffeicola);

Rape seed diseases:
sclerotinia rot (Sclerotinia sclerotiorum), gray leaf spot (Alternaria brassicae), root rot (Phoma lingam), and light leaf spot (Pyrenopeziza brassicae);

Sugar cane diseases:
rust (Puccinia melanocephela and Puccinia kuehnii), and smut (Ustilago scitaminea);

Sunflower diseases:
rust (Puccinia helianthi) and downy mildew (Plasmopara halstedii);

Citrus diseases:
melanose (Diaporthe citri), scab (Elsinoe fawcetti), green mold (Penicillium digitatum), blue mold (Penicillium italicum), Phytophthora rot (Phytophthora parasitica and Phytophthora citrophthora), and aspergillus rot (Aspergillus niger);

Apple diseases:
blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata and Colletotrichum acutatum), blotch (Diplocarpon mali), ring rot (Botryosphaeria berengeriana), crown rot (Phytophtora cactorum), and rust (Gymnosporangium juniperi-virginianae and Gymnosporangium yamadae);

Pear diseases:
scab (Venturia nashicola and Venturia pirina), black spot (Alternaria alternata Japanese pear pathotype), and rust (Gymnosporangium haraeanum);

Peach diseases:
brown rot (Monilinia fructicola), scab (Cladosporium carpophilum), Phomopsis rot (Phomopsis sp.), leaf curl (Taphrina deformans), and powdery mildew (Podosphaera leucotricha);

Grapes diseases:
anthracnose (Elsinoe ampelina), ripe rot (Glomerella cingulata and Colletotrichum acutatum), powdery mildew (Uncinula necator), rust (Neophysopella sp.), black rot (Guignardia bidwellii), downy mildew (Plasmopara viticola), leaf blight (Pseudocercospora vitis), and white rot (Coniella castaneicola);

Japanese persimmon diseases:
anthracnose (Gloeosporium kaki and Colletotrichum acutatum), leaf spot (Cercospora kaki and Mycosphaerella nawae), and powdery mildew (Phyllactinia kakicola);

Fig disease:
rust (Phakopsora nishidana);

Diseases of gourd family:
anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), damping-off (Pythium sp.), and Black root rot (Phomopsis sp.);

Tomato diseases:
early blight (Alternaria solani), leaf mold (Cladosporium fulvum), Cercospora leaf mold (Pseudocercospora fuligena), late blight (Phytophthora infestans), and powdery mildew (Leveillula taurica);

Eggplant diseases:
brown spot (Phomopsis vexans), powdery mildew (Erysiphe cichoracearum), black blight (Corynespora melongenae), leaf mold (Mycovellosiella nattrassii), and brown leaf spot (Thanatephorus cucumeris);

Cruciferous vegetables diseases:
alternaria leaf spot (Alternaria japonica), white spot (Cercosporella brassicae), clubroot (Plasmodiophora brassicae), downy mildew (Peronospora parasitica), white rust (Albugo candida), and Alternaria sooty spot (Alternaria brassicicola);

Welsh onion diseases:
rust (Puccinia allii), black spot (Alternaria porri), white rot (Sclerotium cepivorum), leaf blight (Botrytis squamosa), leaf spot (Stemphylium vesicarium and Stemphylium botryosum), southern blight (Sclerotium rolfsii), leaf blight (Botrytis squamosa);

Soybean diseases:
purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines and Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla, Diaporthe phaseolorum, and Phomopsis longicolla);

Kidney bean diseases:
stem rot (Sclerotinia sclerotiorum), rust (Uromyces appendiculatus), angular leaf spot (Phaeoisariopsis griseola), anthracnose (Colletotrichum lindemuthianum), and Fusarium root-rot (Fusarium solani);

Peanut diseases:
leaf spot (Cercospora personata), brown leaf spot (Cercospora arachidicola), southern blight (Sclerotium rolfsii), and Cylindrocladium black rot (Calonectria ilicicola);

Garden pea diseases:
powdery mildew (Erysiphe pisi) and root rot (Fusarium solani);

Potato diseases:
early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, and Verticillium nigrescens), dry rot (Fusarium solani), and potato wart (Synchytrium endobioticum);

Strawberry diseases:
powdery mildew (Sphaerotheca humuli) and crown rot (Glomerella cingulata and Colletotrichum acutatum);

Tea diseases:
net blister blight (Exobasidium reticulatum), white scab (Elsinoe leucospila), gray blight (Pestalotiopsis sp.), and anthracnose (Colletotrichum theae-sinensis);

Tobacco diseases:
brown spot (Alternaria longipes), anthracnose (Colletotrichum tabacum), blue mold (Peronospora tabacina), and black shank (Phytophthora nicotianae);

Sugar beet diseases:
cercospora leaf spot (Cercospora beticola), leaf blight (Thanatephorus cucumeris), root rot (Thanatephorus cucumeris), aphanomyces root rot (Aphanomyces cochlioides), and rust (Uromyces betae);

Rose diseases:
black spot (Diplocarpon rosae) and powdery mildew (Sphaerotheca pannosa);

Chrysanthemum diseases:
leaf blight (Septoria chrysanthemi-indici) and white rust (Puccinia horiana);

Onion diseases:
botrytis leaf blight (Botrytis cinerea, Botrytis byssoidea, and Botrytis squamosa), gray-mold neck rot (Botrytis allii), and small sclerotial neck rot (Botrytis squamosa);

Various crops diseases:
Botrytis rot (Botrytis cinerea), sclerotinia rot (Sclerotinia sclerotiorum), white rot (Sclerotium cepivorum), southern blight (Sclerotium rolfsii), and seedling blight (Pythium aphanidermatum, Pythium irregulare, and Pythium ultimum);

Japanese radish disease:
alternaria leaf spot (Alternaria brassicicola);

Sweet potato diseases:
stem rot (Fusarium oxysporum f. sp. batatas), foot rot (Diaporthe destruens), and black rot (Ceratocystis fimbriata);

Turfgrass diseases:
dollar spot (Sclerotinia homoeocarpa), brown patch and large patch (Rhizoctonia solani), pythium blight (Pythium aphanidermatum), and anthracnose (Colletotrichum caudatum);

Banana disease:
Sigatoka disease (Mycosphaerella fijiensis and Mycosphaerella musicola);

Lentils disease:
ascochyta blight (Ascochyta lentis);

Chickpea disease:
ascochyta blight (Ascochyta rabiei);

Green pepper diseases:
anthracnose (Colletotrichum scovillei), powdery mildew (Leveillula taurica), and southern blight (Sclerotium rolfsii);

Mango disease:
anthracnose (Colletotrichum acutatum);

ocarina disease:
foot rot (Diaporthe destruens);

Fruit trees diseases:
white root rot (Rosellinia necatrix) and violet root rot (Helicobasidium mompa);

Postharvest disease of fruits (for example, apple and pear) :
Mucor rot diseases (Mucor piriformis);

Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp., or the like;

Viral diseases:
Lettuce big-vein disease transmitted by Olpidium brassicae, and viral diseases of several crops transmitted by Polymyxa spp. (e.g., Polymyxa betae and Polymyxa graminis);

Diseases caused by bacteria:
bacterial seedling blight of rice (Burkholderia plantarii), bacterial palea browning of rice (Pantoea ananatis), Bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), black leg of potato (Pectobacterium carotovorum and Dickeya sp.), bacterial leaf spot of peach (Pseudomona syringae, Erwinia nigrifluens, and Xanthomonas campestris), bacterial canker of Japanese apricot (Prunus mume) (Pseudomonas syringae pv. morsprunorum and Erwinia sp.), Goss's wilt of corn (Clavibacter michiganensis), Pierce's disease of grapes, olive, peach, and the like (Xylella fastidiosa), and crown gall of Rosaceae plants such as apple, peach, and cherries (Agrobacterium tumefaciens);
and the others.

Examples of the Present method for controlling a plant disease include foliage application, application to plant bodies such as seed disinfection, and application to area for cultivating plants such as soil treatment.

The amount of the Present compound to be applied is usually 1 g to 10,000 g per 1,000 m² of area for cultivating plants. When the Present compound is formulated into an emulsifiable concentrate, a wettable powder, a flowable, or the like, the Present compound is usually applied after dilution with water so that the concentration of the active ingredient is controlled within a range of 0.01 to 10,000 ppm. A granule, a dust formulation, or the like is usually applied as it is without being diluted.

The Present composition for controlling a plant disease can be used as an agent for controlling plant diseases in agricultural lands such as fields, paddy fields, lawns, and orchards.

### EXAMPLES

Hereinafter, the present invention is illustrated more in detail by a Preparation Example, Formulation Examples, a Test Example, and the like, but the present invention is not limited to these Examples only.

First, a Preparation Example of the Present compound is shown below.

In the following Example, quantitative analysis was carried out by using high performance liquid chromatography, unless otherwise described. The analysis conditions are as follows.

### [Analysis conditions of high performance liquid chromatography]

Mobile phase: Solution A: 5 mM aqueous solution of ammonium carbonate, Solution B: acetonitrile
Column: SUMIPAX (registered trademark) ODS Z-CLUE, particle size: 3 µm, 4.6 mm I.D. × 100 mm
UV measurement wavelength: 210 nm
Flow rate: 1.0 mL/min
Column oven: 30°C
Pump: two LC-20AD (manufactured by Shimadzu Corporation) (high-pressure gradient)
Gradient conditions: sending a solution with the concentration gradient described in Table LC1.

**Table LC1**

| Time (min) | Solution A (%) | Solution B (%) |
|---|---|---|
| 0 | 65 | 35 |
| 35 | 20 | 80 |
| 50 | 20 | 80 |

### Preparation Example 1

2-(Methylsulfonyl)acetophenone (1.05 g), 3-methyl-3-buten-2-one (454.1 mg), sodium carbonate (28.1 mg), and water (10 mL) were mixed, and the resulting mixture was stirred at room temperature for 20 minutes. The mixture was analyzed by high performance liquid chromatography to confirm that the Present compound was produced with the peak area percentage of 7% (retention time: 9.3 min).

The values of ¹H-NMR of the Present compound are shown below. ¹H-NMR (CDCl₃) δ: 7.54-7.45 (4H, m), 7.40-7.36 (1H, m), 4.65 (1H, d), 3.86 (1H, dd), 2.88-2.84 (1H, m), 2.74 (1H, ddd), 2.68-2.60 (2H, m), 2.28 (1H, dd), 2.01 (3H, s), 1.23 (3H, d).

2-(Methylsulfonyl)acetophenone is commercially available, or may also be produced by using known method(s).

Next, Formulation Examples of the Present compound are shown below. The "part(s)" represents "part(s) by weight".

### Formulation Example 1

A mixture of polyoxyethylene alkyl ether sulfate ammonium salt and silica (weight ratio of 1:1) (35 parts), the Present compound (10 parts), and water (55 parts) are mixed, and the resulting mixture is subjected to fine grinding according to a wet grinding method to obtain a formulation.

### Formulation Example 2

The Present compound (50 parts), calcium lignin sulfonate (3 parts), sodium lauryl sulfate (2 parts), and silica (45 parts) are ground and mixed to obtain a formulation.

### Formulation Example 3

The Present compound (5 parts), polyoxyethylene styryl phenyl ether (9 parts), polyoxyethylene decyl ether (number of added ethylene-oxide: 5) (5 parts), calcium dodecylbenzene sulfonate (6 parts), and xylene (75 parts) are mixed to obtain a formulation.

### Formulation Example 4

The Present compound (2 parts), silica (1 part), calcium lignin sulfonate (2 parts), bentonite (30 parts), and kaolin clay (65 parts) are ground and mixed, an appropriate amount of water is added thereto, the resulting mixture is kneaded, subjected to granulation with a granulator, and then dried to obtain a formulation.

Further, the control efficacy of the Present compound against plant diseases is shown by a Test Example.

The untreated well represents a test well in which the procedures were carried out under the same conditions as those described in the Test Example except that dimethyl sulfoxide was dispensed in place of a diluted dimethyl sulfoxide solution containing the Present compound.

### Test Example 1 Control test against seedling blight fungus (Pythium ultimum)

The Present compound was diluted with dimethyl sulfoxide to obtain a diluted solution containing 500 ppm of the compound, 1 µL of the diluted solution was dispensed into a titer plate (96 well), and thereto was then dispensed 150 µL of a potato dextrose broth medium (PDB medium) to which spores of Pythium ultimum were inoculated in advance. This plate was cultured at 23°C for 5 days, thereby allowing Pythium ultimum to undergo proliferation, and the absorbance at 550 nm of each well of the titer plate was then measured to obtain a value as a degree of growth of Pythium ultimum. As a result, the degree of growth in the well treated with the Present compound was 50% or less relative to the degree of growth in the untreated well.

### INDUSTRIAL APPLICABILITY

The Present compound has excellent control effects against plant diseases, and is useful as an active ingredient of a composition for controlling plant diseases.

## Claims

1. A compound represented by formula (A)

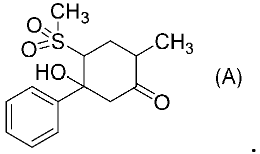

2. A composition for controlling a plant disease comprising the compound according to claim 1 and an inert carrier.

3. A method for controlling a plant disease which comprises applying the compound according to claim 1 or the composition for controlling a plant disease according to claim 2 to a plant or soil for cultivating a plant.
